# EUROPÄISCHE PATENTSCHRIFT

(11) **EP 0 795 752 B1**
(45) Veröffentlichungstag und Bekanntmachung des Hinweises auf die Patenterteilung: **10.10.2001**
(21) Anmeldenummer: 96890047.2
(22) Anmeldetag: 13.03.1996
(51) Int. Cl.: G01N 33/543, G01N 33/553, G01N 33/58, G01N 21/64

(54) **Optochemisches Verfahren und Messanordnung zur Messung der Konzentration eines Analyten**
Optochemical method and device to determine the concentration of an analyte
Procédé et dispositif optochimique pour déterminer la concentration d'un analyte

(43) Veröffentlichungstag der Anmeldung: 17.09.1997
(73) Patentinhaber: F.HOFFMANN-LA ROCHE AG, 4070 Basel (CH)
(72) Erfinder: Karpf, Hellfried, Dr., 8043 Graz (AT); Kirchmayer, Gerald, Dipl.-Ing., 8042 Graz (AT); Muntean, Georg, Dipl.-Ing., 8045 Graz (AT)
(74) Vertreter: Babeluk, Michael, Dipl.-Ing. Mag.

(56) Entgegenhaltungen:
- EP-A- 0 178 083
- EP-A- 0 539 968
- EP-A- 0 677 738
- WO-A-80/02076
- WO-A-88/01058
- WO-A-90/06503
- WO-A-93/02362
- WO-A-95/18376
- US-A- 4 174 384
- R.F. MASSEYEFF ET AL. (EDS.): "Methods of immunological analysis, Volume 1, Fundamentals" , VCH VERLAGSGESELLSCHAFT MBH , WEINHEIM, DE XP002008282 * Seite 227 - Seite 257 * * Seite 297 - Seite 329 *

## Beschreibung

Die Erfindung betrifft eine optochemische Messanordnung mit einem Lumineszenzsensor mit einer biorekognitiven Schicht zur Messung der Konzentration zumindest eines Analyten in einer Probe, wobei der Sensor zumindest eine Inselschicht aufweist, deren Inseln aus elektrisch leitendem Material bestehen und die biorekognitive Schicht auf oder mittels einer Zwischenschicht an der Inselschicht angeordnet ist, wobei weiters ein erstes lumineszierendes System vorgesehen ist, welches den zu messenden Analyten selektiv bindet. Die Erfindung betrifft weiters ein Verfahren zur Messung der Konzentration zumindest eines Analyten in einer Probe.

Optochemische Sensoren bzw. optochemische Messverfahren basieren darauf, dass eine chemische Reaktion zwischen dem optischen Indikator und dem Analyten zu einer Veränderung der optischen Eigenschaften des Sensors führt. Eine Veränderung der optischen Eigenschaften kann beispielsweise in einer Änderung der Absorptions- bzw. der Lumineszenzeigenschaften liegen, sodass die Analytkonzentration durch spektroskopische Methoden nachweisbar wird.

Optische Sensoren zur Messung chemischer Stoffkonzentrationen gewinnen zunehmend an Interesse, da sie gegenüber herkömmlichen Messeinrichtungen wesentlich kürzere Ansprechzeiten, eine größere mechanische Robustheit und eine Unempfindlichkeit gegenüber elektromagnetischen Störfeldern aufweisen.

Aus der GB-A 2 243 683 ist beispielsweise ein optochemischer Sensor bekannt geworden, welcher am Ende einer Fiberoptik eine biorekognitive Schicht aufweist, die mit einem Analyten einer Probe in Kontakt treten kann. Die biorekognitive Schicht weist an Antikörpern gebundene fluoreszenzmarkierte Antigene auf, welche bei Probenkontakt durch den Analyten ersetzt werden. Die abnehmende Fluoreszenz wird als Maß für die Analytkonzentration detektiert.

In der US-A 5 449 918 wird ein optochemischer Sensor zur kontinuierlichen Bestimmung chemischer Spezies beschrieben. Der Sensor basiert auf der Verwendung der Surface-Plasmon-Resonance zur Verstärkung der Fluoreszenzstrahlung chemisch selektiver Membranen, welche als Lang-muir-Blodgett Film auf Inselfilme aufgetragen werden. Die Inselschicht ist ihrerseits auf einem festen Substrat, beispielsweise Glas, aufgetragen.

Die WO-A 93 02362 offenbart eine Oberfläche, welche in analytischen Prozessen eingesetzt werden kann. Die Oberfläche beinhaltet eine metallische Inselschicht sowie eine Zwischenschicht aus einem Kupplungsreagenz, welche die Inselschicht bedeckt und and die Moleküle wie Proteine, Nukleinsäuren, Lipide etc. gebunden werden können. Die Inseln, beispielsweise aus Silber, haben eine Dicke von 2 bis 20 nm und einen Durchmesser von bis zu 14 nm. Ein Assay gemäß WO-A 93 02362 besteht aus einem Partner A, der an eine Oberfläche gebunden ist, einem Partner B, welcher an A bindet und einem Partner C, welcher beispielsweise fluoreszenzmarkiert ist und an B bindet. Die Fluoreszenz eines an die Inselschicht gebundenen Moleküls erhöht sich signifikant im Vergleich zu ungebundenen Molekülen.

Aus der WO-A 95 18376 ist ein Verfahren zur Bestimmung der Konzentration eines Analyten bekannt geworden, wobei der Analyt an einen Rezeptor gebunden wird. Danach werden zwei unterschiedlich markierte Liganden zugesetzt, wobei einer der Liganden spezifisch an den gebundenen Analyten, der andere an unbesetzte Rezeptoren bindet. Aus den dabei erhaltenen relativen Signalen wird auf die Besetzung der Rezeptoren und damit auf die Konzentration des Analyten rückgeschlossen. Dabei können fluoreszierende Marker, wie z.B. Fluorescein oder Coumarin verwendet werden.

Schwierigkeiten ergeben sich bei den bekannten Messanordnungen bzw. Messverfahren dadurch, dass oft Probleme mit erhöhter Hintergrundstrahlung bzw. nicht stabilen Messsignalen aufgrund sich ändernder optischer Anregungs- und Messbedingungen bzw. alternder Sensorbestandteile auftreten.

Aufgabe der Erfindung ist es, optische Messanordnungen bzw. Messverfahren der eingangs beschriebenen Art derart weiterzubilden, dass bereits geringe Stoffkonzentrationen von beispielsweise Antikörpern oder Enzyme in besonders einfacher und reproduzierbarer Weise erfasst werden können. Weiters soll dabei die bei optischen Messanordnungen vorteilhafte, geringe Ansprechzeit auch für die zu beschreibenden, neuen Messanordnungen bzw. Messverfahren gültig sein.

Diese Aufgabe wird erfindungsgemäß dadurch gelöst, dass die biorekognitive Schicht sowohl den zu messenden Analyten sowie ein zweites der Probe zusetzbares oder im Sensor vorliegendes lumineszierendes System zumindest indirekt zu binden vermag, wobei sich das Emissionsspektrum zumindest eines der beiden lumineszierenden Systeme in der Nähe der Inselschicht signifikant ändert, derart,
- dass entweder zumindest eines der beiden lumineszierenden Systeme zwei gekoppelte Moleküle aufweist, von welchen zumindest eines ein Luminophor ist und das andere die Lumineszenz des Luminophors löscht, wobei die Lumineszenzlöschung in der Nähe der Inselschicht aufgehoben ist;
- oder dass zumindest eines der beiden lumineszierenden Systeme einen Luminophor aufweist, dessen Moleküle eine hohe räumliche Dichte aufweisen, welche zu starker Eigenquenchung führt, wobei die Eigenquenchung in der Nähe der Inselschicht aufgehoben ist;
- oder dass zumindest eines der beiden lumineszierenden Systeme zwei räumlich eng benachbarte Moleküle aufweist, welche über gemeinsame Anregungs- und Emissionsspektren verfügen, wobei sich die spektrale Verteilung der Emissionsstrahlung in der Nähe der Inselschicht signifikant ändern; sowie dass eine Einrichtung zur Erfassung der Intensität der Lumineszenzstrahlung beider lumineszierender Systeme vorgesehen ist.

Die erfindungsgemäße Messanordnung zeichnet sich vor allem durch die Verwendung von zwei lumineszierenden Systemen aus, wobei sich bei zumindest einem der beiden Systeme die optischen Eigenschaften in der Nähe der Inselschicht signifikant ändern. Zum Unterschied zur eingangs genannten US-A 5 449 918 befindet sich der Luminophor nicht in einer chemisch selektiven Membran gebunden, sondern ein lumineszierendes System bindet selektiv den zu messenden Analyten, welcher an der biorekognitiven Schicht andockt und so das lumineszierende System in die Nähe der Inselschicht bringt, wodurch sich dessen Emissionsspektrum messbar ändert.

In konventionellen Lumineszenzsensoren müssen Luminophore mit hoher Quantenausbeute eingesetzt werden, um die nötige Empfindlichkeit des Sensors zu gewährleisten. Dabei bewirken jedoch gelöste fluoreszierende Moleküle in der Umgebung des Sensors einen starken Signalhintergrund. Daher muss bei vielen dieser Systeme zur Erlangung eines brauchbaren Signals der Überschuss an gelösten Luminophoren vor der Messung vom eigentlichen Sensor entfernt werden.

Ein erfindungsgemäßes Verfahren zur Messung der Konzentration zumindest eines Analyten in einer Probe zeichnet sich somit dadurch aus,
a) dass die Probe mit einer biorekognitiven Schicht eines Sensors in Kontakt gebracht wird, welche auf oder in unmittelbarer Umgebung zumindest einer Inselschicht mit Inseln aus elektrisch leitendem Material angeordnet ist,
b) dass die Probe mit einem ersten und einem zweiten lumineszierenden System in Kontakt gebracht wird,
c) dass eine Bindung des ersten lumineszierenden Systems an den Analyten sowie die Bindung des Analyten an die biorekognitive Schicht ermöglicht wird,
d) dass eine Bindung des zweiten lumineszierenden Systems an die biorekognitive Schicht ermöglicht wird, wobei zumindest eines der beiden lumineszierenden Systeme zwei gekoppelte Moleküle aufweist, von welchen zumindest eines ein Luminophor ist und das andere die Lumineszenz des Luminophors löscht, wobei die Lumineszenzlöschung in der Nähe der Inselschicht aufgehoben wird und sich das Emissionsspektrum zumindest eines der beiden lumineszierenden Systeme signifikant ändert,
e) dass eine zur Anregung des ersten und zweiten lumineszierenden Systems geeignete Anregungsstrahlung in die zumindest eine Inselschicht eingestrahlt wird,
f) dass die vom ersten und zweiten lumineszierenden System emittierte Lumineszenzstrahlung erfasst und aus den gewonnenen Messwerten auf die Analytkonzentration geschlossen wird.

Von den beiden lumineszierenden Systemen können somit zwei Signale ermittelt werden, wovon eines die durch den Analyten besetzten Kontaktstellen der biorekognitiven Schicht und Messwert, welcher weitgehend unabhängig von den optischen Anregungs- und Detektionsbedingungen auf die Analytkonzentration schließen lässt

Es ist jedoch auch möglich eine Spektralanalyse der emittierten Lumineszenzstrahlung zumindest eines lumineszierenden Systems durchzuführen und aus den spektralen Änderungen auf die Analytkonzentration zu schließen.

In einer Variante des erfindungsgemäßen Verfahrens ist in Punkt d) vorgesehen, dass zumindest eines der beiden lumineszierenden Systeme einen Luminophor aufweist, dessen Moleküle eine hohe räumliche Dichte aufweisen, welche zu starker Eigenquenchung führt, wobei die Eigenquenchung in der Nähe der Inselschicht aufgehoben wird und sich das Emissionsspektrum zumindest eines der beiden lumineszierenden Systeme signifikant ändert, oder dass zumindest eines der beiden lumineszierenden Systeme zwei räumlich eng benachbarte Moleküle aufweist, welche über gemeinsame Anregungs- und Emissionsspektren verfügen, wodurch die spektrale Verteilung der Emissionsstrahlung in der Nähe der Inselschicht signifikant geändert wird

Es hat sich gezeigt, dass die charakteristischen Messeffekte bei einem Lumineszenzsensor der erfindungsgemäßen optochemischen Messanordnung nur im Bereich der energetischen Kopplung (weniger als 20 nm Abstand) der optisch aktiven Luminophore mit der Inselschicht auftreten- Durch die erfindungsgemäße Ausgestaltung mit überaus dünnen Sensorschichten kann ein entsprechend kurzer Diffusionsweg vorgegeben und dadurch die Ansprechzeit des Sensors entsprechend verkürzt werden Mit konventionellen interferometrischen oder Surface Plasmon Resonance-Methoden lassen sich hingegen geringfügige chemische Änderungen in dünnen Schichten nur mit großem messtechnischem Aufwand erfassen.

Weiters kann beispielsweise zur Anregung der ersten und zweiten lumineszierenden Systeme die Inselschicht auf einer transparenten Oberfläche aufgebracht sein, welche zur Einkopplung des Anregungsstrahles (z. B. Laser oder LED) verwendet wird. Zur Anregung kann dabei - wie an sich aus der WO-A 90 06503 bekannt - der evaneszente Anteil des Anregungslichtes verwendet werden.

Besonders starke Änderungen des Emissionsspektrums treten dann auf, wenn die Inseln einen Durchmesser aufweisen, welcher deutlich kleiner als die Wellenlänge der Anregungsstrahlung ist und das Absorptionsminimum mit dem Emissionsmaximum des Luminophors überlappt. Bevorzugt kann hierbei die Ausgestaltung so getroffen werden, dass die Inseln einen Durchmesser von kleiner 100 nm, vorzugsweise kleiner 60 nm bei Verwendung von sichtbarem Licht aufweisen.

Obwohl sich prinzipiell viele Metalle (z. B. auch Aluminium, Nickel und Kupfer) für die Ausbildung der Inselschicht eignen, können vorteilhafterweise nur jene eingesetzt werden, die auch den chemischen Angriffen des Messmediums standhalten. Dazu zählen vor allem Gold und Silber, welche sich darüber hinaus durch besonders vorteilhafte Absorptionseigenschaften und damit verbundene Erhöhung des Messeffektes auszeichnen.

In einer bevorzugten Messgeometrie der optochemischen Messanordnung erfolgt die Einstrahlung des Anregungslichtes von der probenabgewandten, transparenten Seite des Sensors und die Detektion der Messstrahlung auf derselben Seite.

Erfindungsgemäß ist vorgesehen, dass die biorekognitive Schicht an oder über der Inselschicht aus Proteinen, Lipiden, Lektinen, Nukleinsäuren oder artifiziellen Liganden besteht Bevorzugt werden dabei Proteine, wie Antikörper, Antigene und Lektine sowie Hormone, DNA und RNA eingesetzt Derartige biorekognitive Schichten zeichnen sich durch eine selektive Bindung des Analyten aus.

Auch die beiden lumineszierenden Systeme können bevorzugt Proteine, wie Antikörper, Antigene und Lektine sowie Hormone, Lipide, DNA und RNA aufweisen, welche mit einem Luminophor markiert sind.

Beispielsweise ist es erfindungsgemäß vorgesehen, dass die lumineszierenden Systeme über zumindest einen lumineszenzmarkierten Antikörper verfügen, welcher einen den eigentlichen Analyten bindenden Detektionsantikörper zu binden vermag. In der untenstehenden Tabelle sind einige Beispiele für lumineszierende Systeme, welche mit bestimmten Antikörpern der biorekognitiven Schicht zusammenwirken, dargestellt:

| **biorekognit. Schicht** | **Lumineszierendes System** | |
|---|---|---|
| Coating Antikörper | Detektionsantikörper | Markierter Antikörper (bzw. Protein G) |
| **Anti-CK-MB** (monoklonal) | **Anti-CK-B** (monoklonal) | **Protein G-Peroxidase** |
| **Anti-CK-BB** (polyklonales Serum, rabbit) | **Anti-CK-MM** (polyklonales Serum, goat) | **Anti-goat-Peroxidase** |
| **Anti-CK-MM** (polyklonales Serum, goat) | **Anti-CK-BB** (polyklonales Serum rabbit) | **Anti-rabbit-Peroxidase** |
| **Anti-CK-MB** (monoklonal) | **Anti-CK-BB** (polyklonales Serum, rabbit) | **Anti-rabbit-Peroxidase** |

In der Tabelle steht CK für Creatinkinase, M für Muscle und B für Brain.
Um eine hinreichend rasche Ansprechzeit und gleichzeitig auch eine ausgeprägte Erhöhung der Lumineszenzintensität zu gewährleisten, wird mit Vorteil die Ausbildung so getroffen, dass die Dicke der immobilisierten, biorekognitiven Schicht kleiner als 20 nm, insbesondere kleiner als 15 nm gewählt wird. Zur Erhöhung der Lumineszenzausbeute soll die Dicke jedoch nicht unter 3 bis 5 nm betragen wobei prinzipiell Schichtdicken von 5 bis 15 nm optimal realisierbar sind.

Erfindungsgemäß ist vorgesehen, dass die beiden lumineszierenden Systeme zumindest einen Fluorophor aus der Gruppe der Triphenylmethanfarbstoffe sowie der homo- und heterozyklischen, aromatischen Kohlenwasserstoffe aufweisen, vorzugsweise
- Amino-Triphenylmethanfarbstoffe: Malachitgrün, Fuchsin, Kristallviolett;
- Phthaleine: Fluorescein, Eosin
- homozyklische, aromatische Kohlenwasserstoffe: Pyrene, Perylene, Benzo(ghi)perylene, Coronene, Anthracene, Phenanthrene;
- heterozyklische, aromatische Kohlenwasserstoffe: Coumarine, Acridine, Carbazole, Qinoline, Triphenylamine, Imidazole, Porphyrine und -ketone, Übergangsmetallkomplexe mit Diimine-Liganden.

Diese Verbindungen können in substituierter oder unsubstituierter Form vorliegen. Bei den Übergangsmetallkomplexen werden bevorzugt die Zentralatome Ruthenium, Platin und Palladium verwendet.

Die verwendete Inselschicht weist vorteilhafterweise eine Massendicke kleiner 20 nm, vorzugsweise kleiner 15 nm auf, wobei für eine besonders hohe Empfindlichkeit der Inselschicht eine Absorption zwischen 40 und 60 % für die jeweils verwendete Wellenlänge des Anregungslichtes angestrebt wird.

Die beiden lumineszierenden Systeme können auf vielfaltige Art und Weise lumineszenzmarkiert sein. So kann beispielsweise zumindest eines der beiden lumineszierenden Systeme zwei gekoppelte Moleküle aufweisen, von welchen zumindest eines ein Luminophor ist und das andere die Lumineszenz des Luminophors löscht, wobei die Lumineszenzlöschung in der Nähe der Inselschicht aufgehoben ist. Weiters kann zumindest eines der beiden lumineszierenden Systeme einen Luminophor aufweisen, dessen Moleküle eine hohe räumliche Dichte aufweist, welche zu starker Eigenquenchung fuhrt, wobei die Eigenquenchung in der Nähe der Inselschicht aufgehoben ist.

Eine besonders vorteilhafte Ausgestaltung der Erfindung sieht vor, dass zumindest eines der beiden lumineszierenden Systeme zwei räumlich eng benachbarte Moleküle aufweist, welche über gemeinsame Anregungs- und Emissionsspektren verfügen, wobei sich die spektrale Verteilung der Emissionsstrahlung in der Nähe der Inselschicht signifikant ändern. Räumlich eng benachbarte Moleküle, welche über gemeinsame Anregungs- und Emissionsspektren verfügen, werden als Exciplex bezeichnet. Das Emissionsspektrum wird einer Spektralanalyse unterzogen, wobei über spektrale Änderungen auf die Analytkonzentration geschlossen werden kann.

Die Erfindung wird im folgenden anhand von schematischen Zeichnungen näher erläutert.

Es zeigen:
Fig. 1 eine erfindungsgemäße Messanordnung in schematischer Darstellung, die
Fig. 2 und 3 Ausführungsvarianten der erfindungsgemäßen Messanordnung, sowie die
Fig. 4 ein Detail der Sensoroberfläche einer optochemischen Messanordnung.

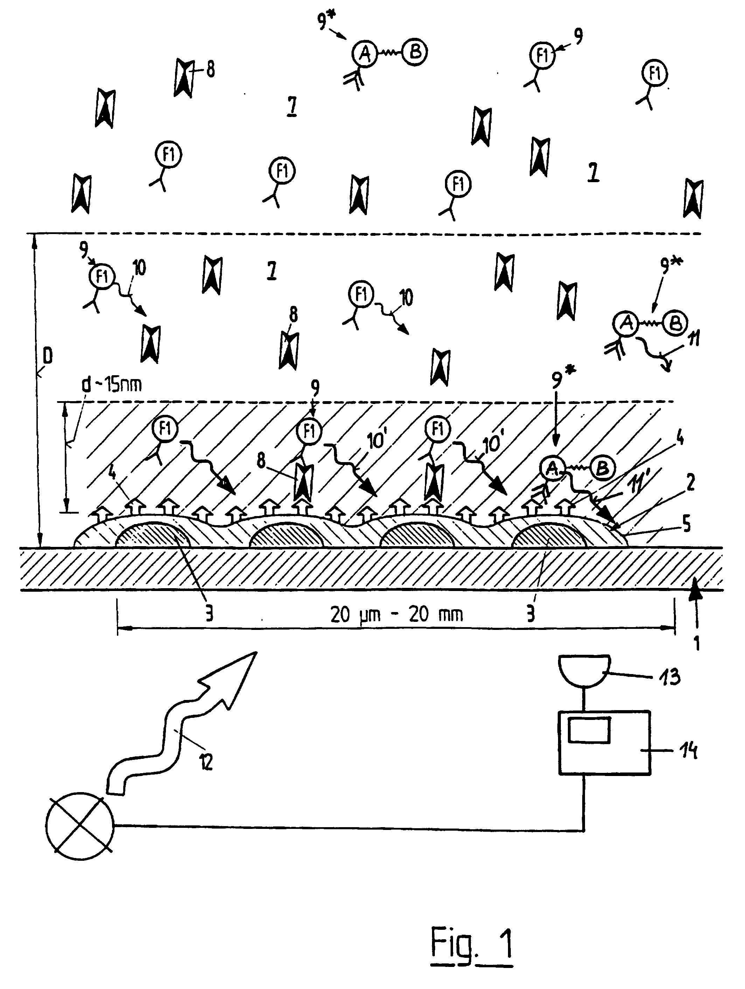

In dem in Fig. 1 dargestellten Beispiel weist die erfindungsgemäße optochemische Messanordnung einen Sensor 1 auf, welcher auf einem für die Anregungs- und Messstrahlung transparenten Substrat eine Inselschicht 2 trägt, die aus einer Vielzahl von elektrisch leitendenden Inseln 3, vorzugsweise aus Gold oder Silber, besteht Die Inseln 3 haben einen Durchmesser kleiner als 300 nm und können beispielsweise auf das Substrat 1 aufgedampft, aufgesputtert oder durch elektronenstrahllithographische Verfahren hergestellt werden. Eine biorekognitive Schicht 4 ist direkt - oder wie in Fig. 1 dargestellt - gebunden über eine Spacer- oder Zwischenschicht 5 mit der Inselschicht 2 verbunden bzw. in deren unmittelbarer Umgebung angeordnet Die biorekognitive Schicht 4 steht direkt oder über eine analytpermeable Membran mit der Probe 7 in Verbindung.

In der Messanordnung ist ein erstes lumineszierendes System 9 vorgesehen, welches den Analyten 8 selektiv zu binden vermag, sowie ein zweites nicht analytspezifisches, lumineszierendes System 9*. Die biorekognitive Schicht 4 des Sensors kann sowohl den Analyten 8 als auch das zweite lumineszierende System 9* binden. Innerhalb der Distanz D, der Eindringtiefe des evaneszenten Anteiles des Anregungslichtes 12, werden die beiden lumineszierenden Systeme 9 und 9* angeregt und emittieren Lumineszenzstrahlung. Die Quantenausbeute zumindest eines der beiden lumineszierenden Systeme 9 und 9* ist außerhalb einer Distanz d von ca. 15 nm (Bereich der Luminophor/Insel-Kopplung) gering (dargestellt durch die Pfeile 10 und 11) und erhöht sich innerhalb der Distanz d stark (siehe Pfeile 10' und 11'). Es ist jedoch auch möglich, dass zumindest eines der beiden lumineszierenden Systeme 9, 9* einen Exciplex bildet, d. h. zwei räumlich eng benachbarte Moleküle aufweist, welche über gemeinsame Anregungs- und Emissionsspektren verfügen. Das Elektronenspektrum des Exciplex verändert sich in der Nähe der Inselschicht wodurch sich auch das Emissionsspektrum signifikant ändert. Die Intensität der Lumineszenzstrahlung beider lumineszierender Systeme 9 und 9* wird von einer Detektionseinrichtung 13 erfasst, wobei in einer Einrichtung 14 eine Spektralanalyse des Emissionsspektrums durchgeführt oder das Verhältnis der beiden Intensitäten ermittelt wird. Die gewonnene Verhältniszahl bzw. spektrale Änderungen sind ein Maß für die Konzentration des Analyten 8 in der Probe 7.

Im dargestellten Beispiel gemäß Fig. 1 weist das erste lumineszierende System 9 ein mit einem Luminophor vorzugsweise Fluorophor F₁ markiertes biorekognitives Molekül auf, welches in der Lage ist, an den Analyten 8 anzukoppeln. Das zweite lumineszierende System 9* verfügt über zwei gekoppelte Luminophore oder Fluorophore A und B, welche ebenfalls ein biorekognitives Molekül markieren, welches jedoch direkt an der biorekognitiven Schicht 4 ankoppelt. Anstelle des gekoppelten Systems der Luminophore A und B könnte auch das zweite lumineszierende System 9* eine einfache Lumineszenzmarkierung aufweisen, es müsste lediglich sichergestellt sein, dass die beiden Luminophore für die Detektions- und Auswerteeinrichtung 13, 14 unterscheidbar sind und zumindest eines der beiden lumineszierenden Systeme 9, 9* eine messbare Änderung in der Nähe der Inselschicht erfährt. Beispielsweise könnte bei der in Fig. 1 dargestellten Ausführungsvariante die Kopplung zwischen den beiden Luminophoren A und B in der Nähe der Inselschicht aufgehoben werden, wodurch sich durch Wegfall der Lumineszenzlöschung die Intensität der Lumineszenastrahlung stark erhöht (siehe Pfeil 11). Beim ersten lumineszierenden System 9 kann in der Nähe der Inselschicht 4 ebenfalls eine Lumineszenzverstärkung auftreten, es könnte jedoch auch ein Luminophor F₁ eingesetzt werden, dessen Intensität sich in der Nähe der Inselschicht nicht ändert, in welchem Fall das lumineszierende System 9 lediglich als Referenzgröße verwendet würde.

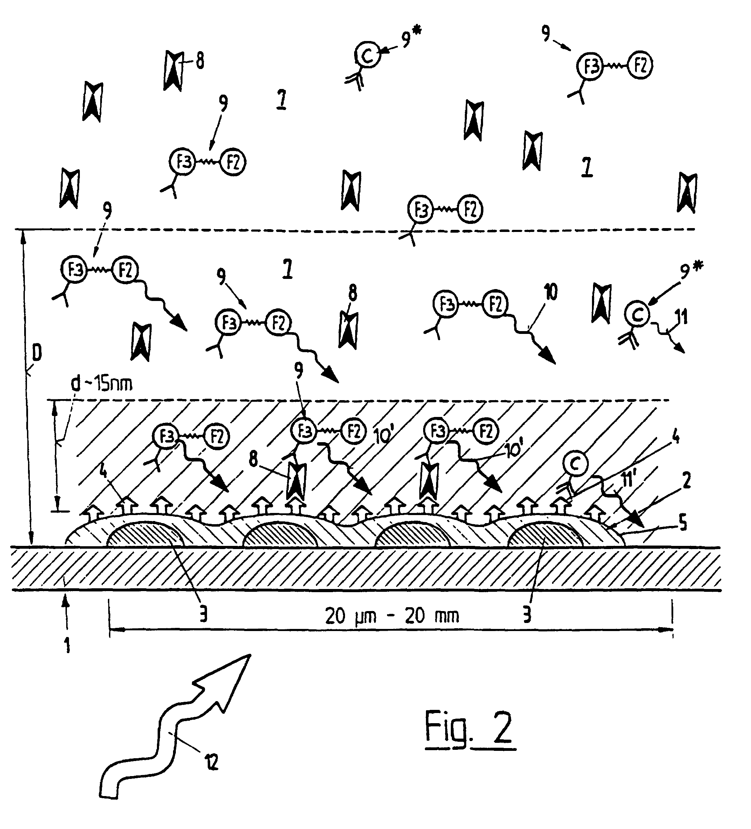

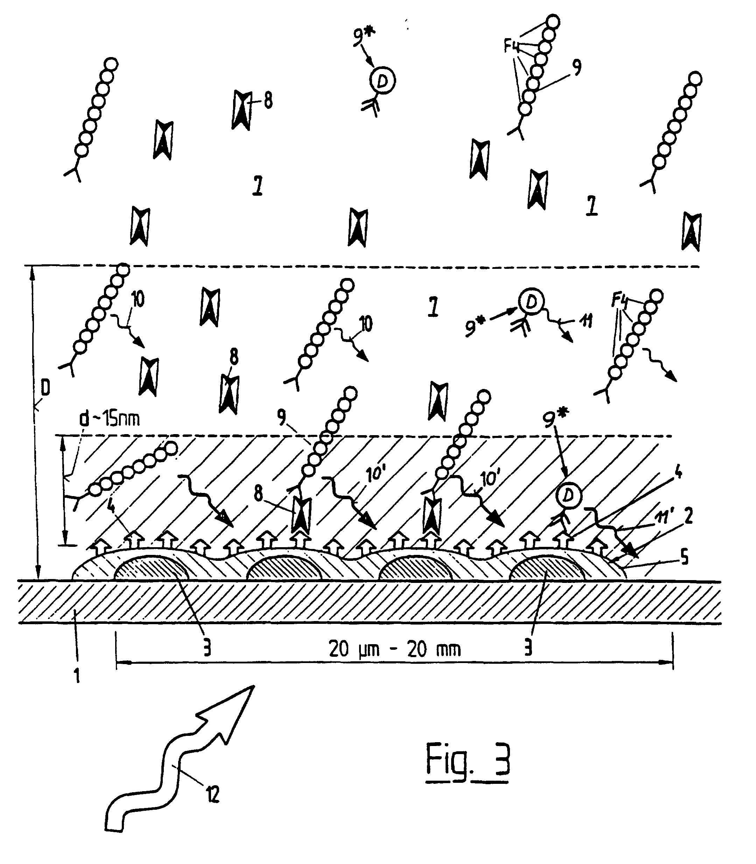

Die Fig. 2 und 3 zeigen im wesentlichen dieselbe Grundstruktur der in Fig. 1 beschriebenen optochemischen Messanordnung, wobei hier allerdings das erste lumineszierende System 9 aus zwei gekoppelten Luminophoren F₂ und F₃ besteht, und das zweite lumineszierende System 9* mit einem Luminophor C markiert ist

In Fig. 3 besteht das lumineszierende System 9 aus einer Vielzahl von Luminophoren F₄, welche eine räumliche Dichte aufweisen, die zu starker Eigenquenchung führt. Die Eigenquenchung wird innerhalb der Distanz d (Bereich der Luminophor, Insel-Kopplung) aufgehoben, sodass die Intensität der Lumineszenzstrahlung ansteigt. Als Referenzgröße kann, ähnlich wie in Fig. 2, ein einfach lumineszenzmarkiertes, biorekognitives Molekül mit einem Luminophor D verwendet werden.

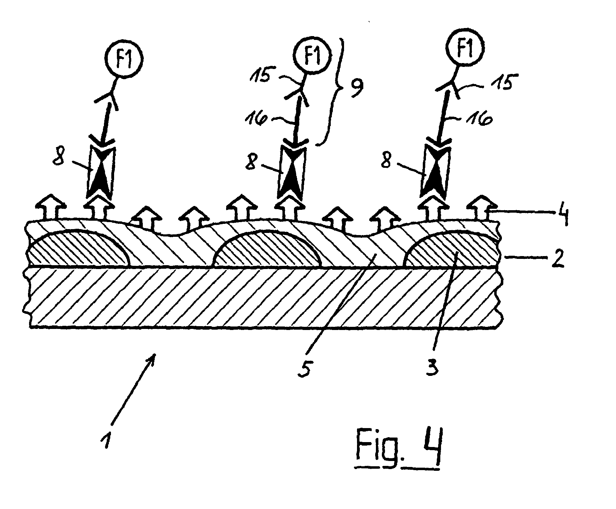

Fig. 4 zeigt einen Ausschnitt aus einem Sensor 1, wobei der Analyt 8 selektiv an Reaktionsstellen der Inselschicht 4 gebunden wird. Das erste lumineszierende System 9 besteht aus einem markierten Antikörper 15, welcher über einen Detektionsantikörper 16 am Analyten 8 andockt. Eine ähnliche Konstruktion ist auch für das zweite lumineszierende System 9* möglich, wobei der Detektionsantikörper bzw. Referenzantikörper über zumindest einen lumineszenzmarkierten Antikörper verfügt und direkt an der biorekognitiven Schicht 4 andockt.

## Patentansprüche

1. Optochemische Messanordnung mit einem Lumineszenzsensor (1) mit einer biorekognitiven Schicht (4) zur Messung der Konzentration zumindest eines Analyten (8) in einer Probe (7), wobei der Sensor (1) zumindest eine Inselschicht (2) aufweist, deren Inseln (3) aus elektrisch leitendem Material bestehen und die biorekognitive Schicht (4) auf oder mittels einer Zwischenschicht (5) an der Inselschicht (2) angeordnet ist, wobei weiters ein erstes lumineszierendes System (9) vorgesehen ist, welches den zu messenden Analyten (8) selektiv bindet, **dadurch gekennzeichnet, dass** die biorekognitive Schicht (4) sowohl den zu messenden Analyten (8) sowie ein zweites der Probe zusetzbares oder im Sensor (1) vorliegendes lumineszierendes System (9*) zumindest indirekt zu binden vermag, wobei sich das Emissionsspektrum zumindest eines der beiden lumineszierenden Systeme (9, 9*) in der Nähe der Inselschicht (2) signifikant ändert, derart,
• dass entweder zumindest eines der beiden lumineszierenden Systeme (9, 9*) zwei gekoppelte Moleküle aufweist, von welchen zumindest eines ein Luminophor ist und das andere die Lumineszenz des Luminophors löscht, wobei die Lumineszenzlöschung in der Nähe der Inselschicht (2) aufgehoben ist;
• oder dass zumindest eines der beiden lumineszierenden Systeme (9, 9*) einen Luminophor aufweist, dessen Moleküle eine hohe räumliche Dichte aufweisen, welche zu starker Eigenquenchung führt, wobei die Eigenquenchung in der Nähe der Inselschicht (2) aufgehoben ist;
• oder dass zumindest eines der beiden lumineszierenden Systeme (9, 9*) zwei räumlich eng benachbarte Moleküle aufweist, welche über gemeinsame Anregungs- und Emissionsspektren verfügen, wobei sich die spektrale Verteilung der Emissionsstrahlung in der Nähe der Inselschicht (2) signifikant ändern;
sowie dass eine Einrichtung (13, 14) zur Erfassung der Intensität der Lumineszenzstrahlung beider lumineszierender Systeme (9, 9*) vorgesehen ist.

2. Messanordnung nach Anspruch 1, **dadurch gekennzeichnet, dass** die biorekognitive Schicht (4) an oder über der Inselschicht (2) aus Proteinen, Lipiden, Lektinen, Nukleinsäuren oder artifiziellen Liganden besteht.

3. Messanordnung nach Anspruch 1 oder 2, **dadurch gekennzeichnet, dass** die lumineszierenden Systeme (9, 9*) über zumindest einen lumineszenzmarkierten Antikörper verfügen, welcher einen den eigentlichen Analyten (8) bindenden Detektionsantikörper zu binden vermag.

4. Messanordnung nach einem der Ansprüche 1 bis 3, **dadurch gekennzeichnet, dass** die beiden lumineszierenden Systeme (9, 9*) zumindest einen Fluorophor aus der Gruppe der Triphenylmethanfarbstoffe sowie der homo- und heterozyklischen, aromatischen Kohlenwasserstoffe aufweisen, vorzugsweise
• Amino-Triphenylmethanfarbstoffe: Malachitgnin, Fuchsin, Kristallviolett;
• Phthaleine: Fluorescein, Eosin;
• homozyklische, aromatische Kohlenwasserstoffe: Pyrene, Perylene, Benzo(ghi)perylene, Coronene, Anthracene, Phenanthrene;
• heterozyklische, aromatische Kohlenwasserstoffe: Coumarine, Acridine, Carbazole, Qinoline, Triphenylamine, Imidazole, Porphyrine und -ketone, Übergangsmetallkomplexe mit Diimine-Liganden.

5. Messanordnung nach einem der Ansprüche 1 bis 4, **dadurch gekennzeichnet, dass** die Inseln (3) der Inselschicht (2) aus Gold oder Silber besteht, und einen Durchmesser kleiner 100 nm, vorzugsweise kleiner 60 nm aufweisen.

6. Verfahren zur Messung der Konzentration zumindest eines Analyten in einer Probe,
**dadurch gekennzeichnet:**
a) dass die Probe mit einer biorekognitiven Schicht eines Sensors in Kontakt gebracht wird, welche auf oder in unmittelbarer Umgebung zumindest einer Inselschicht mit Inseln aus elektrisch leitendem Material angeordnet ist,
b) dass die Probe mit einem ersten und einem zweiten lumineszierenden System in Kontakt gebracht wird,
c) dass eine Bindung des ersten lumineszierenden Systems an den Analyten sowie die Bindung des Analyten an die biorekognitive Schicht ermöglicht wird,
d) dass eine Bindung des zweiten lumineszierenden Systems an die biorekognitive Schicht ermöglicht wird, wobei zumindest eines der beiden lumineszierenden Systeme zwei gekoppelte Moleküle aufweist, von welchen zumindest eines ein Luminophor ist und das andere die Lumineszenz des Luminophors löscht, wobei die Lumineszenzlöschung in der Nähe der Inselschicht aufgehoben wird und sich das Emissionsspektrum zumindest eines der beiden lumineszierenden Systeme signifikant ändert,
e) dass eine zur Anregung des ersten und zweiten lumineszierenden Systems geeignete Anregungsstrahlung in die zumindest eine Inselschicht eingestrahlt wird,
f) dass die vom ersten und zweiten lumineszierenden System emittierte Lumineszenzstrahlung erfasst und aus den gewonnenen Messwerten auf die Analytkonzentration geschlossen wird.

7. Verfahren zur Messung der Konzentration zumindest eines Analyten in einer Probe,
**dadurch gekennzeichnet:**
a) dass die Probe mit einer biorekognitiven Schicht eines Sensors in Kontakt gebracht wird, welche auf oder in unmittelbarer Umgebung zumindest einer Inselschicht mit Inseln aus elektrisch leitendem Material angeordnet ist,
b) dass die Probe mit einem ersten und einem zweiten lumineszierenden System in Kontakt gebracht wird,
c) dass eine Bindung des ersten lumineszierenden Systems an den Analyten sowie die Bindung des Analyten an die biorekognitive Schicht ermöglicht wird,
d) dass eine Bindung des zweiten lumineszierenden Systems an die biorekognitive Schicht ermöglicht wird, wobei zumindest eines der beiden lumineszierenden Systeme einen Luminophor aufweist, dessen Moleküle eine hohe räumliche Dichte aufweisen, welche zu starker Eigenquenchung führt, wobei die Eigenquenchung in der Nähe der Inselschicht aufgehoben wird und sich das Emissionsspektrum zumindest eines der beiden lumineszierenden Systeme signifikant ändert,
e) dass eine zur Anregung des ersten und zweiten lumineszierenden Systems geeignete Anregungsstrahlung in die zumindest eine Inselschicht eingestrahlt wird,
f) dass die vom ersten und zweiten lumineszierenden System emittierte Lumineszenzstrahlung erfasst und aus den gewonnenen Messwerten auf die Analytkonzentration geschlossen wird.

8. Verfahren zur Messung der Konzentration zumindest eines Analyten in einer Probe,
**dadurch gekennzeichnet:**
a) dass die Probe mit einer biorekognitiven Schicht eines Sensors in Kontakt gebracht wird, welche auf oder in unmittelbarer Umgebung zumindest einer Inselschicht mit Inseln aus elektrisch leitendem Material angeordnet ist,
b) dass die Probe mit einem ersten und einem zweiten lumineszierenden System in Kontakt gebracht wird,
c) dass eine Bindung des ersten lumineszierenden Systems an den Analyten sowie die Bindung des Analyten an die biorekognitive Schicht ermöglicht wird,
d) dass eine Bindung des zweiten lumineszierenden Systems an die biorekognitive Schicht ermöglicht wird, wobei zumindest eines der beiden lumineszierenden Systeme zwei räumlich eng benachbarte Moleküle aufweist, welche über gemeinsame Anregungs- und Emissionsspektren verfügen, wodurch die spektrale Verteilung der Emissionsstrahlung in der Nähe der Inselschicht signifikant geändert wird,
e) dass eine zur Anregung des ersten und zweiten lumineszierenden Systems geeignete Anlegungsstrahlung in die zumindest eine Inselschicht eingestrahlt wird,
f) dass die vom ersten und zweiten lumineszierenden System emittierte Lumineszenzstrahlung erfasst und aus den gewonnenen Messwerten auf die Analytkonzentration geschlossen wird.

9. Verfahren nach einem der Ansprüche 6 bis 8, **dadurch gekennzeichnet, dass** in Punkt e) die Anregung des ersten und zweiten lumineszierenden Systems mit dem evaneszenten Anteil der Anregungsstrahlung erfolgt.

10. Verfahren nach einem der Ansprüche 6 bis 9, **dadurch gekennzeichnet, dass** in Punkt f) die Lumineszenzintensität beider lumineszierender Systeme erfasst und aus dem Verhältnis beider Lumineszenzintensitäten die Analytkonzentration bestimmt wird.

11. Verfahren nach einem der Ansprüche 6 bis 9, **dadurch gekennzeichnet, dass** in Punkt f) eine Spektralanalyse der emittierten Lumineszenzstrahlung zumindest eines lumineszierenden Systems durchgeführt und aus den spektralen Änderungen auf die Analytkonzentration geschlossen wird.

## Claims

1. Optical chemical measuring device comprising a luminescence sensor 1 with a biorecognitive layer 4 for measuring the concentration of at least one analyte 8 in a sample 7, where the sensor 1 has at least one island film 2 whose islands 3 consist of electrically conductive material, the bio-recognitive layer 4 being deposited on the island film 2 or bound thereto via an intermediate layer 5, and where a first luminescent system 9 is provided, which selectively binds the analyte 8 being measured, **characterised in that** the biorecognitive layer **4** is capable of binding, at least indirectly, both the analyte 8 being measured and a second luminescent system 9* to be added to the sample or being present in the sensor 1, the emission spectrum of at least one of the two luminescent systems 9, 9* changing significantly near the island film 2, such that
• at least one of the two luminescent systems 9, 9* exhibits two coupled molecules, at least one of which is a luminophore, while the other one quenches the luminescence of the luminophore, no luminescence quenching taking place near the island film 2, or
• at least one of the two luminescent systems 9, 9* exhibits a luminophore whose molecules have high spatial density leading to strong self-quenching, no self-quenching taking place near the island film 2, or
• at least one of the two luminescent systems 9, 9* exhibits two molecules in close spatial proximity, which have joint excitation and emission spectra, the spectral distribution of the emission radiation changing significantly near the island film 2,
and that a unit 13, 14 is provided for detection of the intensity of the luminescence radiation of both luminescent systems 9, 9*.

2. A measuring device according to claim 1, **characterised in that** the biorecognitive layer 4 on or above the island film 2 consists of proteins, lipids, lectines, nucleic acids, or artifical ligands.

3. A measuring device according to claim 1 or 2, **characterised in that** the luminescent systems 9, 9* include at least one luminescence-labelled antibody capable of binding a detection antibody which in turn binds the actual analyte 8.

4. A measuring device according to any of claims 1 to 3, **characterised in that** the two luminescent systems 9, 9* exhibit at least one fluorophore from the groups of triphenylmethane dyes and homocyclic and heterocyclic, aromatic hydrocarbons, preferably
amino-triphenylmethane dyes: malachite green, fuchsine, crystal violet;
phthaleins: fluorescein, eosin;
homocyclic, aromatic hydrocarbons: pyrenes, perylenes, benzo(ghi)perylenes, coronenes, anthracenes, phenanthrenes;
heterocyclic, aromatic hydrocarbons: coumarins, acridines, carbazoles, quinolines, triphenylamines, imidazoles, porphyrins and -ketones, transition metal complexes with diimine ligands.

5. A measuring device according to any of claims 1 to 4, **characterised in that** the islands 3 of the island film 2 consist of gold or silver and have a diameter smaller than 100 nm, and preferably smaller than 60 nm.

6. A method of measuring the concentration of at least one analyte in a sample, **characterised in that**
(a) the sample is contacted with a biorecognitive sensor layer, which is deposited on or in close vicinity of at least one island film comprising islands of electrically conductive material,
(b) the sample is contacted with a first and a second luminescent system,
(c) the first luminescent system is enabled to bind to the analyte, which analyte in turn binds to the biorecognitive layer,
(d) the second luminescent system is enabled to bind to the biorecognitive layer, where at least one of the two luminescent systems exhibits two coupled molecules, at least one of which is a luminophore, while the other one quenches the luminescence of the luminophore, no luminescence quenching taking place near the island film 2, and where the emission spectrum of at least one of the two luminescent systems changes significantly,
(e) an excitation radiation suitable for exciting the first and second luminescent system is radiated into the at least one island film,
(f) the luminescence radiation emitted by the first and second luminescent system is detected and the measured values are used to determine the analyte concentration.

7. A method of measuring the concentration of at least one analyte in a sample, **characterised, in that**
(a) the sample is contacted with a biorecognitive sensor layer , which is deposited on or in close vicinity of at least one island film comprising islands of electrically conductive material,
(b) the sample is contacted with a first and a second luminescent system,
(c) the first luminescent system is enabled to bind to the analyte, which analyte in turn binds to the biorecognitive layer,
(d) the second luminescent system is enabled to bind to the biorecognitive layer, where at least one of the two luminescent systems exhibits a luminophore, whose molecules have high spatial density leading to strong self-quenching, no self-quenching taking place near the island film, and where the emission spectrum of at least one of the two luminescent systems changes significantly,
(e) an excitation radiation suitable for exciting the first and second luminescent system is radiated into the at least one island film,
(f) the luminescence radiation emitted by the first and second luminescent system is detected and the measured values are used to determine the analyte concentration.

8. A method of measuring the concentration of at least one analyte in a sample, **characterised in that**
(a) the sample is contacted with a biorecognitive sensor layer , which is deposited on or in close vicinity of at least one island film comprising islands of electrically conductive material,
(b) the sample is contacted with a first and a second luminescent system,
(c) the first luminescent system is enabled to bind to the analyte, which analyte in turn binds to the biorecognitive layer,
(d) the second luminescent system is enabled to bind to the biorecognitive layer, where at least one of the two luminescent systems exhibits two molecules in close spatial proximity, which have joint excitation and emission spectra, the spectral distribution of the emission radiation changing significantly near the island film,
(e) an excitation radiation suitable for exciting the first and second luminescent system is radiated into the at least one island film,
(f) the luminescence radiation emitted by the first and second luminescent system is detected and the measured values are used to determine the analyte concentration.

9. A method according to any of claims 6 to 8, **characterised in that** in (e) excitation of the first and second luminescent system is effected via the evanescent portion of the excitation radiation.

10. A method according to any of claims 6 to 9, **characterised in that** in (f) the luminescence intensities of both luminescent systems are detected, the ratio of the two luminescence intensities providing a measure of the analyte concentration.

11. A method according to any of claims 6 to 9, **characterised in that** in (f) a spectral analysis is performed of the luminescence emission from at least one luminescent system, the spectral changes providing a measure of the analyte concentration.

## Revendications

1. Dispositif de mesure optochimique comprenant un détecteur de luminescence (1) avec une couche biosélective (4) pour mesurer la concentration d'au moins un analyte (8) dans un échantillon (7), où le détecteur (1) comporte au moins une couche (2) à îles dont les îles (3) sont constituées d'un matériau conducteur d'électricité, où la couche biosélective (4) est disposée sur la couche (2) à îles via une couche intermédiaire (5), où on prévoit un premier système luminescent (9) qui se fixe sélectivement à l'analyte à déterminer, **caractérisé en ce que** la couche biosélective (4) permet la fixation au moins indirecte non seulement de l'analyte (8) à déterminer, mais également d'un second système luminescent (9*) qui peut être ajouté à l'échantillon ou qui est présent dans le détecteur (1), **en ce que** le spectre d'émission d'au moins un des deux systèmes luminescents (9, 9*) est modifié de manière significative au voisinage de la couche (2) à îles et **en ce que** :
• soit au moins un des deux systèmes luminescents (9, 9*) présente deux molécules couplées dont au moins une est un luminophore et l'autre provoque une extinction de la luminescence du luminophore, l'extinction de la luminescence étant inhibée au voisinage de la couche (2) à îles,
• soit au moins un des deux systèmes luminescents (9, 9*) présente un luminophore dont la molécule présente une haute densité spatiale, ce qui augmente l'effet d'autoextinction, l'autoextinction étant inhibée au voisinage de la couche (2) à îles,
• soit au moins un des deux systèmes luminescents (9, 9*) présente deux molécules spatialement très proches ayant des spectre d'excitation et d'émission communs, la répartition spectrale du rayonnement d'émission étant modifiée de manière significative au voisinage de la couche (2) à îles,
ainsi qu'un dispositif (13, 14) pour mesurer l'intensité des rayonnements luminescents des deux systèmes luminescents (9, 9*).

2. Dispositif de mesure selon la revendication 1, **caractérisé en ce que** la couche biospécifique (4) sur la couche (2) à îles est constituée de protéines, de lipides, de lectines, d'acides nucléiques ou de ligands artificiels.

3. Dispositif de mesure selon la revendication 1 ou la revendication 2, **caractérisé en ce que** le système luminescent (9, 9*) comporte au moins un anticorps marqué pour être luminescent qui est capable de se fixer à un anticorps de détection se fixant à l'analyte (8) proprement dit.

4. Dispositif de mesure selon l'une des revendications 1 à 3, **caractérisé en ce que** les deux systèmes luminescents (9, 9*) présentent au moins un fluorophore choisi dans le groupe des colorants du type triphénylméthane, ainsi que des hydrocarbures aromatiques homocycliques et hétérocycliques, la préférence allant aux
• colorants du type aminotriphénylméthane, vert de malachite, fuchsine, violet cristal,
• phthaléine, fluorescéine, éosine,
• colorants hydrocarbonés homocycliques aromatiques : pyrène, pérylène, benzo(ghi)pérylène, coronène, anthracène et phénanthrène,
• colorants hydrocarbonés hétérocycliques aromatiques : coumarine, acridine, carbazole, quiloléine, triphénylamine, imidazole, porphyrine et -cétone, complexes de métaux de transition avec des ligands diimine.

5. Dispositif de mesure selon l'une des revendications 1 à 4, **caractérisé en ce que** les îles (3) de la couche (2) à îles sont constituées d'or ou d'argent et qu'elles ont un diamètre inférieur à 100 nm et, de préférence, inférieur à 60 nm.

6. Méthode pour mesurer la concentration en au moins un analyte dans un échantillon, **caractérisée en ce que :**
a) l'échantillon est amené en contact avec une couche biosélective d'un détecteur qui est en contact direct ou au voisinage immédiat d'au moins une couche à îles dont les îles sont réalisées en un matériau électriquement conducteur,
b) l'échantillon est amené en contact avec un premier et un second systèmes luminescents,
c) le premier système luminescent se fixe sur l'analyte et l'analyte se fixe sur la couche biosélective,
d) le second système luminescent se fixe sur la couche biosélective, au moins un des deux systèmes luminescents comprend deux molécules couplées, dont au moins une est un luminophore et l'autre provoque une extinction de la luminescence du luminophore, l'extinction de la luminescence est inhibée au voisinage de la couche à îles et le spectre d'émission d'au moins un des systèmes luminescents est modifié de manière significative,
e) un rayonnement d'excitation spécifique est émis vers la ou les couches à îles pour exciter le premier et le second systèmes luminescents,
f) le rayonnement émis par luminescence du premier et du second systèmes luminescents est capté et la valeur de mesure obtenue est convertie en mesure de la concentration de l'analyte.

7. Méthode pour mesurer la concentration d'au moins un analyte dans un échantillon, **caractérisée en ce que :**
a) l'échantillon est amené en contact avec une couche biosélective d'un détecteur qui est en contact direct ou au voisinage immédiat d'au moins une couche à îles dont les îles sont réalisées en un matériau électriquement conducteur,
b) l'échantillon est amené en contact avec un premier et un second systèmes luminescents,
c) le premier système luminescent se fixe sur l'analyte et l'analyte se fixe sur la couche biosélective,
d) le second système luminescent se fixe sur la couche biosélective, au moins un des deux systèmes luminescents présente un luminophore, dont les molécules présentent une densité spatiale élevée, ce qui conduit à une auto-extinction augmentée, cette auto-extinction est inhibée au voisinage de la couche à îles et le spectre d'émission d'au moins un des deux systèmes luminescents est modifié de manière significative,
e) un rayonnement d'excitation spécifique est émis vers la ou les couches à îles pour exciter le premier et le second systèmes luminescents,
f) le rayonnement émis par luminescence du premier et du second systèmes luminescents est capté et la valeur de mesure obtenue est convertie en mesure de la concentration de l'analyte.

8. Méthode pour mesurer la concentration d'au moins un analyte dans un échantillon, **caractérisée en ce que :**
a) l'échantillon est amené en contact avec une couche biosélective d'un détecteur qui est en contact direct ou au voisinage immédiat d'au moins une couche à îles dont les îles sont réalisées en un matériau électriquement conducteur,
b) l'échantillon est amené en contact avec un premier et un second systèmes luminescents,
c) le premier système luminescent se fixe sur l'analyte et l'analyte se fixe sur la couche biosélective,
d) le second système luminescent se fixe sur la couche biosélective, au moins un des deux systèmes luminescents présente deux molécules spatialement très proches, qui présentent les mêmes spectres d'excitation et d'émission, et une modification significative de la distribution du spectre d'émission se produit au voisinage de la couche à îles,
e) un rayonnement d'excitation spécifique est émis vers la ou les couches à îles pour exciter le premier et le second systèmes luminescents,
f) le rayonnement émis par luminescence du premier et du second systèmes luminescents est capté et la valeur de mesure obtenue est convertie en mesure de la concentration de l'analyte.

9. Méthode selon l'une des revendications 6 à 8, **caractérisée en ce qu'**à l'étape e), l'excitation du premier et du second systèmes luminescents se fait par la partie évanescente des rayonnements d'excitation.

10. Méthode selon l'une des revendications 6 à 9, **caractérisée en ce qu'**à l'étape f), on capte la luminescence des deux systèmes luminescents et la concentration en analyte est déterminée à partir du rapport des intensités des deux luminescences.

11. Méthode selon l'une des revendications 6 à 9, **caractérisée en ce qu**'à l'étape f) on effectue une analyse spectrale des rayonnements de luminescence émis par au moins un système luminescent et que la modification spectrale est utilisée pour déterminer la concentration en analyte.
